# EUROPEAN PATENT APPLICATION

(11) **EP 3 427 680 A1**
(43) Date of publication of application: **16.01.2019**
(21) Application number: 18182345.1
(22) Date of filing: 09.07.2018
(51) Int. Cl.: A61B 18/08, A61B 18/14, A61B 18/00, A61B 17/00, A61B 17/32, A61B 18/12

(54) **ENERGY-BASED SURGICAL DEVICES FACILITATING BREAKDOWN OF TISSUE SPECIMENS FOR REMOVAL**

(30) Priority: 10.07.2017 US 201715645276
(71) Applicant: Covidien LP, Mansfield, MA 02048 (US)
(72) Inventor: BEGG, Nikolai, D., Wayland, MA Massachusetts 01778 (US); PRIOR, Scott, J., Shelton, CT Connecticut 06484 (US)
(74) Representative: Maschio, Antonio

(57) **Abstract**

A surgical device includes a shaft, first and second end effectors extending distally from the shaft in spaced-apart relation relative to one another to define an area therebetween, and a resection member configured for positioning at least partially within the area defined between the first and second end effectors. Each of the first and second end effectors includes opposing portions movable relative to one another and configured to grasp tissue therebetween. The resection member is configured to extend distally beyond the first and second end effectors, is selectively energizable, and is configured to resect tissue grasped between the first and second end effectors.

## Description

### BACKGROUND

### Technical Field

The present disclosure relates to tissue specimen removal and, more particularly, to energy-based devices facilitating breakdown of tissue specimens to enable removal from an internal body cavity.

### Background of Related Art

In minimally-invasive surgical procedures, operations are carried out within an internal body cavity through small entrance openings in the body. The entrance openings may be natural passageways of the body or may be surgically created, for example, by making a small incision into which a cannula is inserted.

Minimally-invasive surgical procedures may be used for partial or total removal of tissue from an internal body cavity. However, the restricted access provided by minimally-invasive openings (natural passageways and/or surgically created openings) presents challenges with respect to removal of large tissue specimens. As such, tissue specimens that are deemed too large for intact removal are broken down into a plurality of smaller pieces to enable removal from the internal body cavity. With respect to breaking down such tissue specimens, there is the challenge of doing so within confines of the internal body cavity.

### SUMMARY

As used herein, the term "distal" refers to the portion that is described which is further from a user, while the term "proximal" refers to the portion that is being described which is closer to a user. Further, any or all of the aspects described herein, to the extent consistent, may be used in conjunction with any or all of the other aspects described herein.

Provided in accordance with aspects of the present disclosure is a surgical device including a shaft, first and second end effectors extending distally from the shaft in spaced-apart relation relative to one another to define an area therebetween, and a resection member configured for positioning at least partially within the area defined between the first and second end effectors. Each of the first and second end effectors includes opposing portions movable relative to one another and configured to grasp tissue therebetween. The resection member is configured to extend distally beyond the first and second end effectors, is selectively energizable, and is configured to resect tissue grasped between the first and second end effectors.

In an aspect of the present disclosure, the resection member is selectively deployable from a retracted position, wherein the resection member is disposed within the shaft, to an extended position, wherein the resection member extends distally from the shaft at least partially within the area defined between the first and second end effectors.

In another aspect of the present disclosure, the resection member, in the retracted position, is disposed in a collapsed condition. In the extended position, the resection member is disposed in an expanded condition.

In yet another aspect of the present disclosure, in the extended position, the resection member defines a height greater than heights of the first and second end effectors so as to extend beyond the first and second end effectors in opposing height directions.

In still another aspect of the present disclosure, the resection member defines a loop configuration including a leading portion.

In another aspect of the present disclosure, the resection member is pivotable relative to the first and second end effectors through an arcuate path defining a diameter greater than heights of the first and second end effectors. In such aspects, the resection member may include a wire defining a semi-circular loop, may include a cup defining a portion of a sphere, or may define an elongated configuration including a proximal end portion about which the resection member is pivotable and a distal end portion including an energizable component.

In another aspect of the present disclosure, each of the first and second end effectors includes first and second jaw members movable relative to one another from a spaced-apart position to an approximated position to grasp tissue therebetween. First and second closure tubes may be provided for moving the first and second jaw members of each of the first and second end effectors from the spaced-apart position to the approximated position. Alternatively, cam-slot mechanisms, coupled with drive rods may be provided to move the first and second jaw members of each of the first and second end effectors from the spaced-apart position to the approximated position.

In yet another aspect of the present disclosure, the resection member is adapted to connect to a source of electrosurgical energy, e.g., monopolar or bipolar electrosurgical energy.

In another aspect of the present disclosure, the resection member is adapted to connect to a source of laser energy.

In still another aspect of the present disclosure, a housing is disposed at a proximal end portion of the shaft. The housing includes at least one actuator configured to manipulate the first and second end effectors for grasping tissue therewith. The housing may additionally or alternatively include at least one second actuator configured to manipulate the resection member. The housing may additionally or alternatively include an activation button configured to selectively energize the resection member.

In yet another aspect of the present disclosure, a robotic arm is disposed at a proximal end portion of the shaft. The robotic arm includes at least one actuator configured to manipulate the first and second end effectors for grasping tissue therewith. Alternatively or additionally, the robotic arm includes at least one second actuator configured to manipulate the resection member.

### BRIEF DESCRIPTION OF THE DRAWINGS

The above and other aspects and features of the present disclosure will become more apparent in light of the following detailed description when taken in conjunction with the accompanying drawings wherein like reference numerals identify similar or identical elements and:
FIG. 1 is a perspective view of an energy-based surgical device provided in accordance with the present disclosure, wherein first and second pairs of jaw members of an end effector assembly of the surgical device are each disposed in a spaced-apart position;
FIG. 2A is a longitudinal, cross-sectional view of a proximal end portion of the surgical device of FIG. 1 with portions removed to illustrate a first drive assembly of the surgical device;
FIG. 2B is a longitudinal, cross-sectional view of the proximal end portion of the surgical device of FIG. 1 with other portions removed to illustrate a second drive assembly of the surgical device;
FIG. 3A is a side view of a distal end portion of the surgical device of FIG. 1, wherein the pairs of jaw members of the end effector assembly are each disposed in an approximated position and a resecting wire of the surgical device is disposed in a retracted position;
FIG. 3B is a side view of the distal end portion of the surgical device of FIG. 1, wherein the pairs of jaw members of the end effector assembly are each disposed in the approximated position and the resecting wire is disposed in a first extended position;
FIG. 3C is a side view of the distal end portion of the surgical device of FIG. 1, wherein the pairs of jaw members of the end effector assembly are each disposed in the approximated position and the resecting wire is disposed in a second extended position;
FIG. 4 is a top view of the distal end portion of the surgical device of FIG. 1 in use grasping and resecting a tissue specimen;
FIG. 5 is a side view of another configuration of a distal end portion configured for use with the energy-based surgical device of FIG. 1;
FIG. 6 is a top view of the distal end portion of FIG. 5;
FIG. 7 is a longitudinal, cross-sectional view of a proximal end portion of another energy-based surgical device provided in accordance with the present disclosure with portions removed to illustrate one of the drive assemblies of the surgical device;
FIG. 8A is a side view of a distal end portion of the surgical device of FIG. 7 with portions removed to illustrate a resecting wand of an end effector assembly of the surgical device, the resecting wand disposed in a first position;
FIG. 8B is a side view of the distal end portion of the surgical device of FIG. 7 with portions removed to illustrate the resecting wand, the resecting wand disposed in a second position;
FIGS. 9A-9C are perspective views of a distal end portion of another energy-based surgical device provided in accordance with the present disclosure illustrating movement of a resecting wire of the surgical device between first, second, and third positions;
FIGS. 10A-10C are side, schematic views illustrating movement of the resecting wire of the surgical device of FIGS. 9A-9C between the first, second, and third positions;
FIGS. 11A-11C are side, schematic views illustrating movement of a resecting cup of another energy-based surgical device provided in accordance with the present disclosure between first, second, and third positions; and
FIG. 12 is a schematic illustration of a robotic surgical system configured for use in accordance with the present disclosure.

### DETAILED DESCRIPTION

The present disclosure provides energy-based surgical devices facilitating breakdown of tissue specimens within an internal body cavity to enable removal from the internal body cavity.

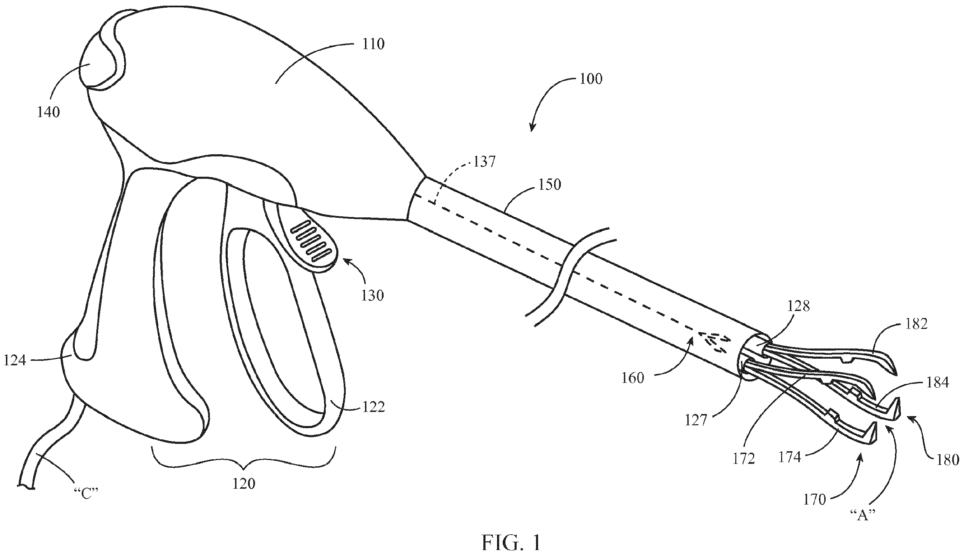

Turning to FIG. 1, an energy-based surgical device 100 provided in accordance with the present disclosure is shown generally including a housing 110, a handle assembly 120 operably coupled to housing 110, a trigger assembly 130 operably coupled to housing 110, an activation button 140 operably coupled to housing 110, a shaft 150 extending distally from the housing 110, a resection wire 160 slidably disposed within shaft 150, and first and second end effectors 170, 180, respectively, operably supported at a distal end portion of shaft 150.

An electrosurgical cable "C" is configured to connect surgical device 100 to a source of electrosurgical energy (not shown) to enable selectively delivery of energy to resection wire 160, e.g., upon activation of activation button 140, as detailed below. Resection wire 160 may be configured to receive monopolar energy and serve as an active electrode for use with a remote return pad (not shown) to conduct energy to tissue to resect tissue. Alternatively, resection wire 160 may define one electrode in a bipolar configuration with an electrically-isolated component(s) of surgical device 100, e.g., one or both of end effectors 170, 180, serving as the other electrode to enable conduction of energy therebetween and through tissue to resect tissue. Alternatively, resection wire 160 may be configured as a resistively-heated element for treating tissue with thermal energy to resect tissue, or may be energizable in any other suitable manner for resecting tissue.

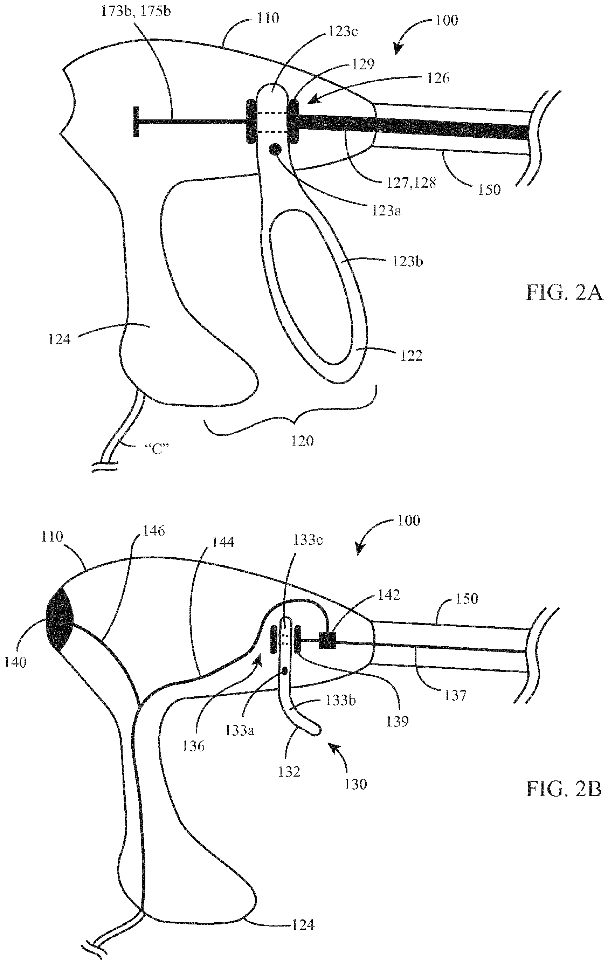

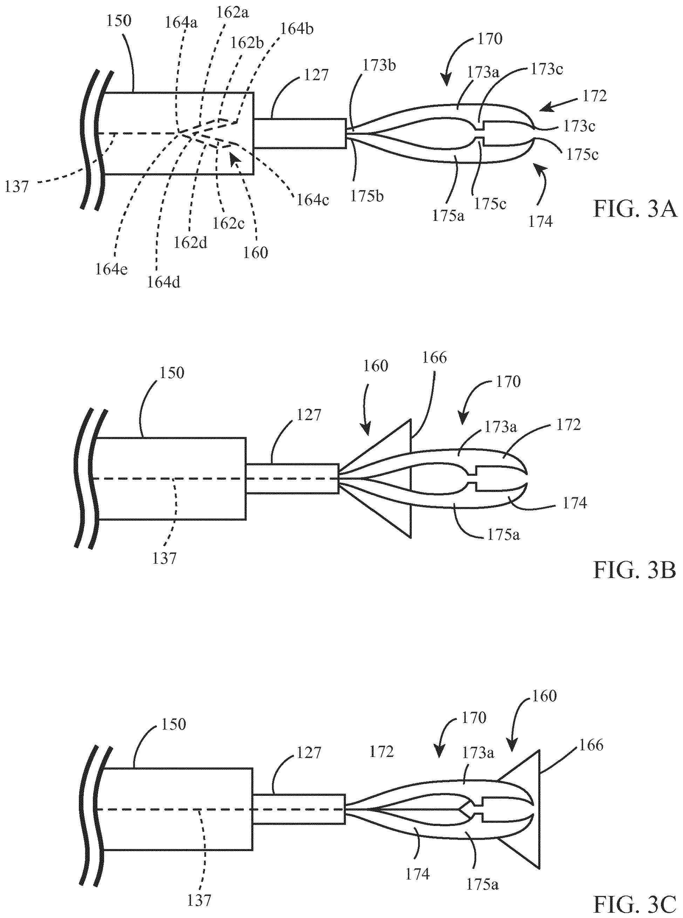

Continuing with reference to FIG. 1, first and second end effectors 170, 180, respectively, extend distally from shaft 150 in laterally spaced-apart relation relative to one another to define an area "A" therebetween. Each of first and second end effectors 170, 180 includes a pair of jaw members 172, 174 and 182, 184, respectively, disposed in opposed relation relative to one another. Jaw members 172, 174 of end effector 170 each include a distal body portion 173a, 175a and an elongated proximal portion 173b, 175b extending proximally from distal body portion 173a, 175a. Distal body portions 173a, 175a of jaw members 172, 174 are resiliently flexible, e.g., formed from spring steel or other suitable material, and biased apart from one another towards a spaced-apart position (FIG. 1). A first closure tube 127 of a first drive assembly 126 (see FIG. 2A) of surgical device 100 is movable distally about and relative to jaw members 172, 174 to urge distal body portions 173a, 175a towards an approximated position (FIGS. 3A-3C), enabling distal body portions 173a, 175a to grasp tissue therebetween. Distal body portions 173a, 175a may include tissue-engagement features 173c, 175c, e.g., teeth, protrusions, etc., configured to facilitate grasping of tissue and inhibit slippage of grasped tissue. The proximal end portions of elongated proximal portions 173b, 175b of jaw members 172, 174 may be fixedly mounted within housing 110 (see FIG. 2A) or otherwise fixed in position to enable first closure tube 127 (FIG. 2A) to slide about and relative to jaw members 172, 174. Alternatively, first closure tube 127 (FIG. 2A) may be fixed and jaw members 172, 174 may slide relative thereto between the spaced-apart position (FIG. 1) and the approximated position (FIGS. 3A-3C).

Jaw members 182, 184 of second end effector 180 are similar to jaw members 172, 174 of first end effector 170 and, thus, are not described in detail hereinbelow. A second closure tube 128 of first drive assembly 126 (see FIG. 2A) is distally movable about and relative to jaw members 182, 184, similarly as detailed above with respect to first closure tube 127 and jaw members 172, 174, to move jaw members 182, 184 from the spaced-apart position to the approximated position.

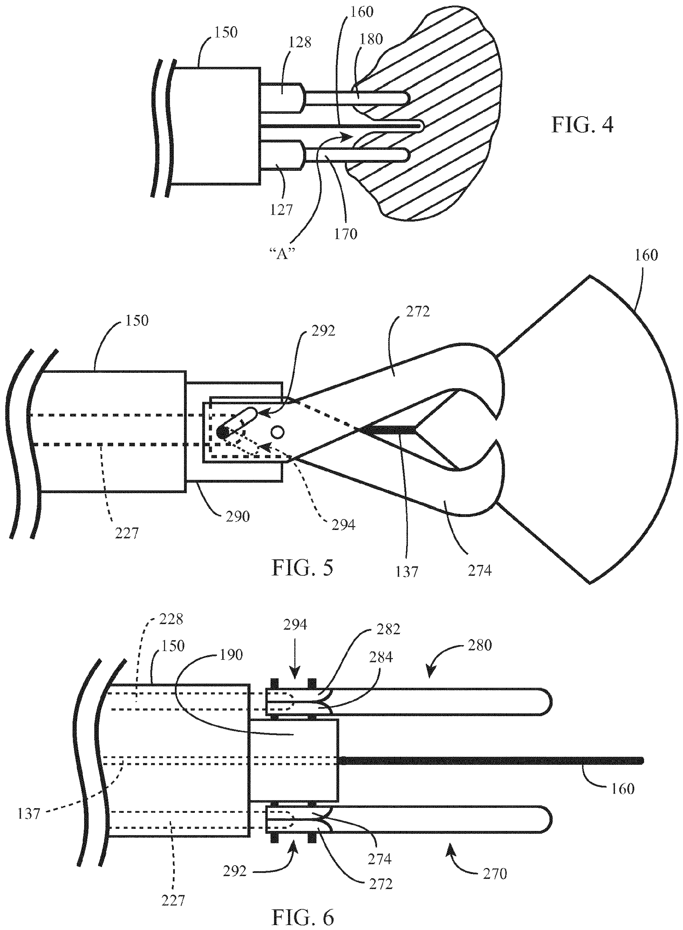

Referring to FIGS. 1 and 3A-3C, resection wire 160 is slidably disposed within shaft 150 and defines a loop configuration having multiple segments 162a-162d interconnected by living hinges 164a-164e, although other configurations are also contemplated. An actuation shaft 137 of second drive assembly 136 (see FIG. 2B) extends through shaft 150 and supports resection wire 160 at a distal end portion thereof. Actuation shaft 137 is slidable through shaft 150 to move resection wire 160 relative to shaft 150 between a retracted position (FIG. 1 and 3A), wherein resection wire 160 is disposed within shaft 150, and one or more extended positions (FIGS. 3B and 3C), wherein resection wire 160 extends distally from shaft 150 into or distally beyond the area "A" defined between end effectors 170, 180. In the retracted position, resection wire 160 is disposed in a collapsed configuration, enabling the looped resection wire 160 to fit within shaft 150. Upon movement of resection wire 160 to one of the extended positions, resection wire 160 resiliently returns to a presented configuration wherein resection wire 160 defines a generally triangular-shaped configuration having a leading portion 166, although other configurations, e.g., an arcuate leading portion (see FIG. 5), are also contemplated. Leading portion 166 of resection wire 160 defines a height greater than that of end effectors 170, 180 such that leading portion 166 extends above and below end effectors 170, 180 in the extended positions thereof. Resection wire 160 is adapted to connect to a source of electrosurgical energy and activation button 140 such that, upon activation of activation button 140, resection wire 160 is energized. With resection wire 160 energized, resection wire 160 may be urged into tissue, lead by leading portion 166 thereof, to resect tissue.

With additional reference to FIG. 2A, handle assembly 120 includes a movable handle 122 and a fixed handle 124 that is integral with or otherwise fixed relative to housing 110. Movable handle 122 is operably coupled to first and second end effectors 170, 180 via a first drive assembly 126 such that pivoting of movable handle 122 relative to fixed handle 124 between an initial position and a compressed position moves the first and second jaw members 172, 174 and 182, 184 of each of first and second end effectors 170, 180, respectively, from the spaced-apart position (FIG. 1) to the approximated position (FIGS. 3A-3C). More specifically, movable handle 122 is pivotably coupled to housing 110 via a pivot pin 123a and includes a grasping portion 123b disposed on one side of the pivot pin 123a and a flange portion 123c disposed on the other side of the pivot pin 123a. Flange portion 123c is coupled to a mandrel 129 of first drive assembly 126 which, in turn, is coupled to each of the first and second closure tubes 127, 128. Alternatively, flange portion 123c may be coupled to a pair of mandrels 129, each of which is coupled to one of the closure tubes 127, 128. As a result of the above-detailed configuration, pivoting of movable handle 122 proximally towards fixed handle 124 from the initial position towards the compressed position urges flange portion 123c and, thus, mandrel 129 distally, thereby moving closure tubes 127, 128 distally to move the first and second jaw members 172, 174 and 182, 184 of end effectors 170, 180, respectively, from the spaced-apart position (FIG. 1) towards the approximated position (FIGS. 3A-3C). In this manner, end effectors 170, 180 may be utilized to grasp tissue on either side of the area "A" such that tissue to be resected is held in position extending across the area "A" (see FIG. 4). A biasing member (not shown) may be provided for biasing movable handle 122 towards the initial position and, thus, jaw members 172, 174 and 182, 184 of end effectors 170, 180, respectively, towards the spaced apart position.

With reference to FIGS. 1, 2B, and 3A-3C, trigger assembly 130 includes a trigger 132 operably coupled to a second drive assembly 136 that, in turn, is operably coupled to resection wire 160 such that pivoting of trigger 132 relative to housing 110 between an un-actuated position and an actuated position moves resection wire 160 from the retracted position (FIG. 3A) to one or more extended positions (FIGS. 3A and 3B). More specifically, trigger 132 is pivotably coupled to housing 110 via a pivot pin 133a and includes a grasping portion 133b disposed on one side of the pivot pin 133a and a flange portion 133c disposed on the other side of the pivot pin 133a. Flange portion 133c is coupled to a mandrel 139 of second drive assembly 136 which, in turn, is coupled to actuation shaft 137. Actuation shaft 137 may be formed from an electrically-conductive material or may include one or more electrical leads (not shown) extending therethrough to transmit electrosurgical energy to resection wire 160. A slip ring contact 142 or other suitable electrical connector is slidably disposed about actuation shaft 137 in electrical communication therewith, or is electrically coupled to the electrical leads (not shown) extending therethrough. Slip ring contact 142, in turn, is electrically coupled to activation button 140 and the source of electrosurgical energy (not shown) via lead wires 144, 146 extending through electrosurgical cable "C" and into housing 110 such that, upon activation of activation button 140, electrosurgical energy is delivered to resection wire 160. A biasing member (not shown) may be provided to bias trigger 132 towards the un-actuated position, thereby biasing resection wire 160 towards the extended positions thereof

As a result of the above-detailed configuration, pivoting of trigger 132 proximally from the un-actuated position towards the actuated position urges flange portion 133c and, thus, mandrel 139 distally, thereby moving actuation shaft 137 distally to move resection wire 160 from the retracted position (FIG. 3A), to a first extended position (FIG. 3B) and, upon further actuation of trigger 132, to a second extended position (FIG. 3C). As noted above, upon movement of resection wire 160 from the retracted position (FIG. 3A) to the first extended position (FIG. 3B), resection wire 160 is transitioned from the collapsed configuration to the presented configuration. Thus, with additional reference to FIG. 4, upon distal advancement of resection wire 160 from the first extended position (FIG. 3B) to the second extended position (FIG. 3C), with resection wire 160 energized, e.g., via activation of activation button 140, leading portion 166 of resection wire 160 is urged into tissue held across area "A" via end effectors 170, 180 to resect tissue. As noted above, resection wire 160 is configured to extend above and below end effectors 170, 180 and may further be configured to extend distally there beyond to enable resection of tissue outside of area "A."

Turning to FIGS. 5 and 6, in conjunction with FIGS. 1 and 2A, in embodiments, rather than providing end effectors 170, 180 including resiliently flexible jaw members 172, 174 and 182, 184, respectively, movable from a spaced-apart position towards an approximated position in response to distal advancement of closure tubes 127, 128, respectively, end effectors 270, 280 may be provided including respective rigid first and second jaw members 272, 274 and 282, 284, pivotably coupled to one another and a clevis 290 extending distally from shaft 150. The first and second jaw members 272, 274 and 282, 284 of each end effector 270, 280, respectively, are operably coupled to a respective drive bar 227, 228 by way of a cam-slot mechanism 292, 294. Drive bars 227, 228 are operably coupled to mandrel 129 of first drive assembly 126 such that pivoting of movable handle 122 proximally towards fixed handle 124 from the initial position towards the compressed position move the first and second jaw members 272, 274 and 282, 284 of end effectors 270, 280, respectively, from the spaced-apart position towards the approximated position. End effectors 270, 280 may otherwise be similar to end effectors 170, 280, detailed above.

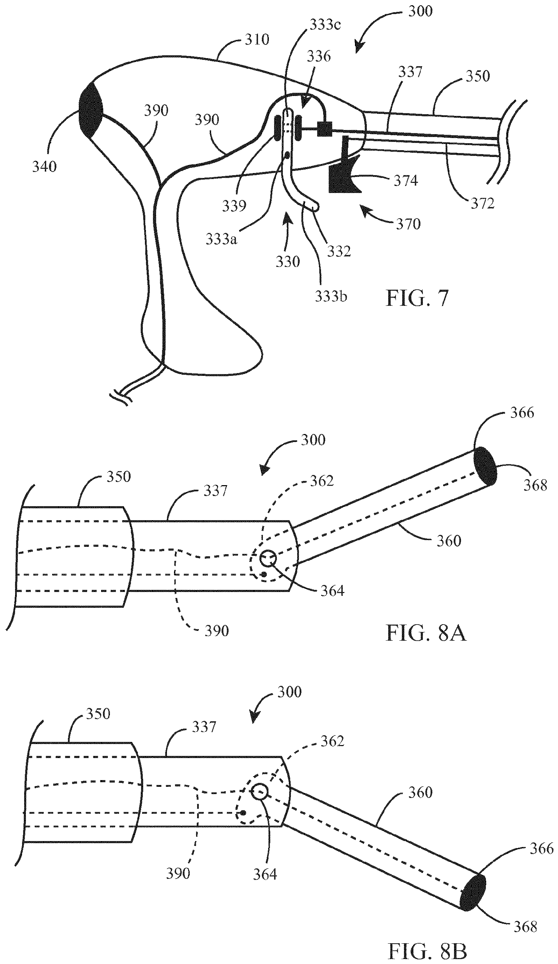

With reference to FIGS. 7-8B, another energy-based surgical device 300 provided in accordance with the present disclosure is shown generally including a housing 310, a trigger assembly 330 operably coupled to housing 310, an activation button 340 operably coupled to housing 310, a shaft 350 extending distally from the housing 310, a resection member 360 slidably and pivotably movable relative to shaft 350, and a slider assembly 370 operably coupled to housing 310. Energy-based surgical device 300 may additionally include a handle assembly (not shown, similar to handle assembly 120 (FIGS. 1 and 2A), a first drive assembly (not shown, similar to first drive assembly 126 (FIG. 2A), and first and second end effectors (not shown, similar to end effectors 170, 180 (FIGS. 1-3C) or end effectors 270, 280 (FIGS. 5-6)) to enable grasping of tissue on either side of resection member 360 to maintain tissue to be resected in position, thus facilitating resection of tissue with resection member 360, similarly as detailed above with respect to surgical instrument 100 (FIG. 1).

Resection member 360 defines a proximal end portion 362 pivotably coupled to a distal end portion of actuation shaft 337 of second drive assembly 336 via a pivot pin 364 and a distal end portion 366 spaced-apart from proximal end portion 362. As detailed below, proximal end portion 362 of resection member 360 is operably coupled to slider assembly 370 to enable selective pivoting of resection member 360 relative to actuation shaft 337 to thereby move distal end portion 366 of resection member 360 through an arcuate path. A proximal end portion of actuation shaft 337 is coupled to trigger 332 of trigger assembly 330. Trigger assembly 330, more specifically, includes a trigger 332 is pivotably coupled to housing 310 via a pivot pin 333a and includes a grasping portion 333b disposed on one side of the pivot pin 333a and a flange portion 333c disposed on the other side of the pivot pin 333a. Flange portion 333c is coupled to a mandrel 339 of second drive assembly 336 which, in turn, is coupled to actuation shaft 337.

As a result of the above-detailed configuration, pivoting of trigger 332 proximally from the un-actuated position towards the actuated position urges flange portion 333c and, thus, mandrel 339 distally, thereby moving actuation shaft 337 distally to move resection member 360 from a retracted position, wherein resection member 360 is at least partially disposed within shaft 350, to an extended position, wherein resection member 360 extends distally from shaft 350. Alternatively, resection member 360 may be longitudinally fixed in the extended position with pivot pin 364 coupled to the distal end portion of shaft 350, thus obviating the need for trigger assembly 330 and second drive assembly 336.

In the extended position of resection member 360, resection member 360 may be pivoted about and relative to actuation shaft 337 to move distal end portion 366 of resection member 360 through an arcuate path, as noted above. In order to enable such movement of resection member 360, slider assembly 370 includes a pull cable 372 operably coupled, at a distal end portion thereof, to proximal end portion 362 of resection member 360 at a location radially-spaced from pivot pin 364. Pull cable 372 is operably coupled, at a proximal end portion thereof, to a slider 374 disposed on housing 310, although other suitable actuators are also contemplated. Slider 374 is movable along housing 310 to pull pull cable 372, thereby urging resection member 360 to pivot such that distal end portion 366 of resection member 360 is moved through the arcuate path from a first position (FIG. 8A) to a second positon (FIG. 8B). Other suitable mechanisms for moving resection member 360 between the first position (FIG. 8A) and the second positon (FIG. 8B) are also contemplated. Further, a biasing member (not shown) may be provided to bias resection member 360 towards the first position (FIG. 8A). Distal end portion 366 of resection member 460 may be configured to move through a path that extends above and below the end effectors used therewith and/or distally beyond the end effectors used therewith.

With continued reference to FIGS. 7-8B, distal end portion 366 of resection member 366 includes an energy emitting component 368. Energy emitting component 368 may include a laser; plasma emitter, e.g., argon plasma emitter; ultrasonic blade; RF electrode (monopolar or bipolar); resistive heater; cryogenic emitter; or other suitable energy emitter. Energy emitting component 368 is coupled to a suitable source of energy (not shown) and activation button 340 of housing 310 via one or more suitable energy transmission components 390. Thus, upon activation of activation button 340, distal end portion 366 of resection member 360 is energized, thus enabling resection of tissue (e.g., grasped tissue, in embodiments where end effectors are provided on either side of resection member 360) as distal end portion 366 of resection member 360 is moved between the first position (FIG. 8A) and the second positon (FIG. 8B).

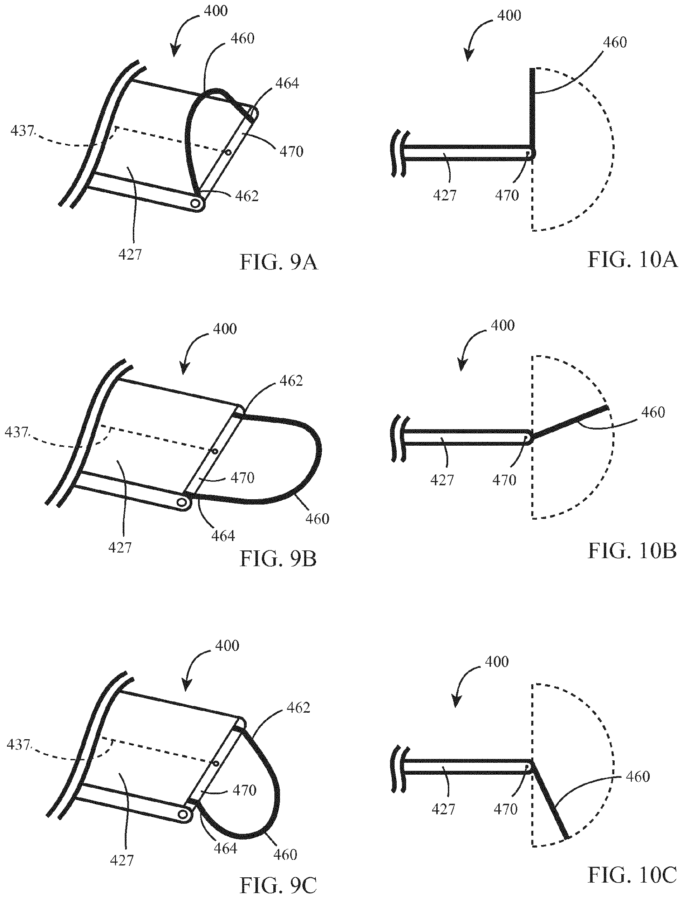

Turning to FIGS. 9A-10C, the distal end portion of another energy-based surgical device 400 provided in accordance with the present disclosure is shown. Surgical device 400 may include any of the features of the energy-based surgical instruments detailed above such as, for example, a housing, a shaft extending distally from the housing, and a pair of spaced-apart end effectors extending distally from the shaft that cooperate to grasp tissue adjacent a distal end portion of the shaft via a handle and first drive assembly.

Surgical device 400 is shown including a drive plate 427, a pull cable 437, a resection wire 460, and a pivot pin 470. Drive plate 427 supports resection wire 460 and pivot pin 470 at a distal end portion thereof. In embodiments, drive plate 427 is configured to extend proximally through a shaft (not shown) and operably couple to a handle assembly (not shown), similarly as detailed above, such that actuation of a movable handle (not shown) of the handle assembly extends and retracts drive plate 427 and, thus, resection wire 460, between retracted and extended positions, similarly as detailed above. Alternatively, drive plate 427 may be fixed in position relative to the shaft.

As noted above, drive plate 427 supports resection wire 460 and pivot pin 470 at a distal end portion thereof. More specifically, pivot pin 470 extends transversely relative to the distal end portion of drive plate 427 and is pivotably coupled thereto at either end portion of pivot pin 470. Resection wire 460 defines a semi-circular configuration (although other configurations are also contemplated) including free ends 462, 464 coupled to pivot pin 470 towards the opposed end portions of pivot pin 470.

A distal end portion of pull cable 437 is coupled to pivot pin 470 at a radially-offset position such that proximal pulling of pull cable 437 rotates pivot pin 470 and, thus, resection wire 460, relative to drive plate 427. A proximal end portion of pull cable 437 is coupled to, for example, a trigger assembly (not shown), similarly as detailed above to enable selective movement of resection wire 460 through a semi-spherical path (see FIGS. 10A-10C), although resection wire 460 may alternatively be configured to move substantially through 360 degrees (except for where drive shaft 427 occupies a portion of the full spherical path), or any suitable range of motion therebetween. Electrical leads (not shown) electrically coupling resection wire 460, an activation button (not shown), and a source of electrosurgical energy (not shown) may be provided, similarly as detailed above, to enable selective energization of resection wire 460 such that, with resection wire 460 energized, resection wire 460 may be moved through the semi-spherical path (see FIGS. 10A-10C) to resect generally spherical sections of tissue from a tissue specimen, e.g., a tissue specimen grasped between end effectors (as detailed above). Resection wire 460 may be configured to move through a path that extends above and below the end effectors used therewith and/or distally beyond the end effectors used therewith. Further, a biasing member(s) may be provided to bias resection wire 460 towards a suitable at-rest position, e.g., the first position (FIGS. 9A and 10A).

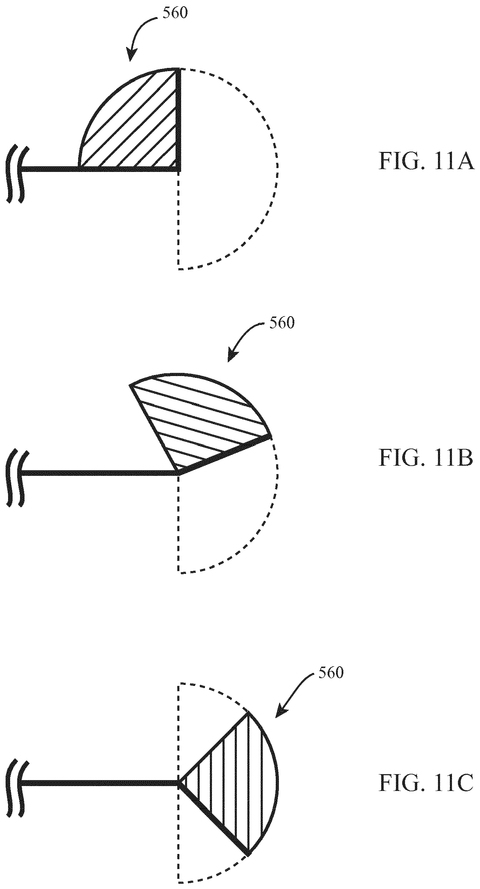

Referring to FIGS. 11A-11C, another resection member in the form of a resection cup 560 configured for use with surgical device 400 (FIGS. 9A-9C) is shown. Resection cup 560 defines a hollow, quarter-spherical configuration and, when energized, similarly as detailed above with respect to resection wire 460 (FIGS. 9A-10C), is movable through a semi-spherical path (see FIGS. 10A-10C), a substantially spherical path, or any suitable range of motion therebetween, to resect generally spherical sections of tissue from a tissue specimen, e.g., a tissue specimen grasped between end effectors (as detailed above). The entire resection cup 560 may serve as an electrode or only select portions thereof may be energizable, e.g., the leading edge, with the remainder being electrically insulated. Resection cup 560 may be configured to move through a path that extends above and below the end effectors used therewith and/or distally beyond the end effectors used therewith.

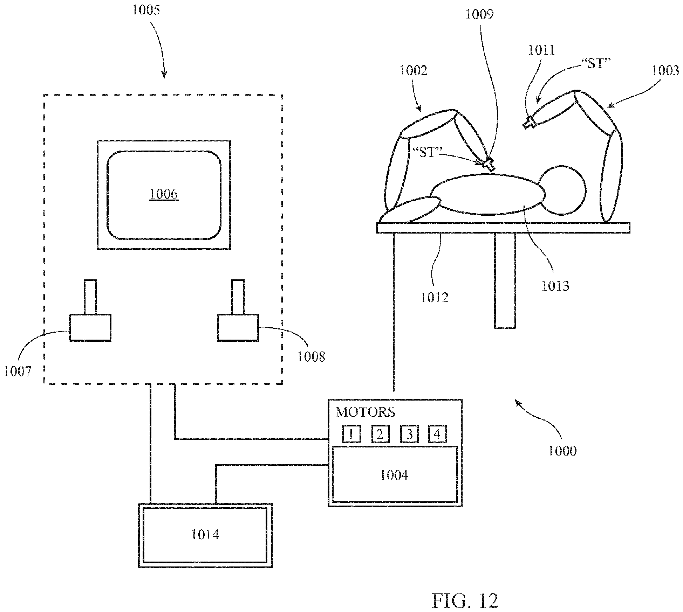

Turning to FIG. 12, as an alternative to manual actuation via a handle assembly, trigger assembly, slider assembly, and/or actuation button, the energy-based surgical devices of the present disclosure may be configured for use with a robotic surgical system 1000 configured to selectively manipulate the end effectors and resection components and to energize the resection components to enable tissue resection. That is, in embodiments, robotic surgical system 1000 may replace handle assembly, trigger assembly, slider assembly, and/or actuation button in favor of utilizing robotic surgical system 1000 to perform what is commonly referred to as "Telesurgery." Robotic surgical system 1000, as detailed below, employs various robotic elements to assist the surgeon and allow remote operation (or partial remote operation). More specifically, various robotic arms, gears, cams, pulleys, electric and mechanical motors, etc. may be employed for this purpose and may be designed with robotic surgical system 1000 to assist the surgeon during the course of an operation or treatment. Robotic surgical system 1000 may include remotely steerable systems, automatically flexible surgical systems, remotely flexible surgical systems, remotely articulating surgical systems, wireless surgical systems, modular or selectively configurable remotely operated surgical systems, etc.

Robotic surgical system 1000 may be employed with one or more consoles that are next to the operating theater or located in a remote location. In this instance, one team of surgeons or nurses may prep the patient for surgery and configure robotic surgical system 1000 with one or more of the instruments disclosed herein while another surgeon (or group of surgeons) remotely control the instruments via the robotic surgical system 1000. As can be appreciated, a highly skilled surgeon may perform multiple operations in multiple locations without leaving his/her remote console which can be both economically advantageous and a benefit to the patient or a series of patients.

The robotic arms of the robotic surgical system 1000 are typically coupled to a pair of master handles by a controller. The handles can be moved by the surgeon to produce a corresponding movement of the working ends of any type of surgical instrument (e.g., end effectors, graspers, knifes, scissors, etc.) which may complement the use of one or more of the embodiments described herein. The movement of the master handles may be scaled so that the working ends have a corresponding movement that is different, smaller or larger, than the movement performed by the operating hands of the surgeon. The scale factor or gearing ratio may be adjustable so that the operator can control the resolution of the working ends of the surgical instrument(s).

The master handles may include various sensors to provide feedback to the surgeon relating to various tissue parameters or conditions, e.g., tissue resistance due to manipulation, cutting or otherwise treating, pressure by the instrument onto the tissue, tissue temperature, tissue impedance, etc. As can be appreciated, such sensors provide the surgeon with enhanced tactile feedback simulating actual operating conditions. The master handles may also include a variety of different actuators for delicate tissue manipulation or treatment further enhancing the surgeon's ability to mimic actual operating conditions.

Referring still to FIG. 12, robotic surgical system 1000, more specifically, includes a plurality of robot arms 1002, 1003; a control device 1004; and an operating console 1005 coupled with control device 1004. Operating console 1005 may include a display device 1006, which may be set up in particular to display three-dimensional images; and manual input devices 1007, 1008, by means of which a person (not shown), for example a surgeon, may be able to telemanipulate robot arms 1002, 1003 in a first operating mode.

Each of the robot arms 1002, 1003 may include a plurality of members, which are connected through joints, and an attaching device 1009, 1011, to which may be attached, for example, a surgical tool "ST" in accordance with any one of several embodiments disclosed hereinabove, or any other suitable surgical tool "ST."

Robot arms 1002, 1003 may be driven by electric drives (not shown) that are connected to control device 1004. Control device 1004 (e.g., a computer) may be set up to activate the drives, in particular by means of a computer program, in such a way that robot arms 1002, 1003, their attaching devices 1009, 1011 and thus the surgical tool "ST" execute a desired movement according to a movement defined by means of manual input devices 1007, 1008. Control device 1004 may also be set up in such a way that it regulates the movement of robot arms 1002, 1003 and/or of the drives.

Robotic surgical system 1000 may be configured for use on a patient 1013 lying on a patient table 1012 to be treated in a minimally invasive manner by means of end effector 1100. Robotic surgical system 1000 may also include more than two robot arms 1002, 1003, the additional robot arms likewise being connected to control device 1004 and being telemanipulatable by means of operating console 1005. A medical instrument or surgical tool may also be attached to the additional robot arm. Robotic surgical system 1000 may include a database 1014, in particular coupled to with control device 1004, in which are stored, for example, pre-operative data from patient/living being 1013 and/or anatomical atlases.

From the foregoing and with reference to the various drawings, those skilled in the art will appreciate that certain modifications can be made to the present disclosure without departing from the scope of the same. While several embodiments of the disclosure have been shown in the drawings, it is not intended that the disclosure be limited thereto, as it is intended that the disclosure be as broad in scope as the art will allow and that the specification be read likewise. Therefore, the above description should not be construed as limiting, but merely as exemplifications of particular embodiments. Those skilled in the art will envision other modifications within the scope and spirit of the claims appended hereto.
The invention may be described by reference to the following numbered paragraphs:-
1. A surgical device, comprising:
   a shaft;
   first and second end effectors extending distally from the shaft in spaced-apart relation relative to one another to define an area therebetween, each of the first and second end effectors including opposing portions movable relative to one another and configured to grasp tissue therebetween; and
   a resection member configured for positioning at least partially within the area defined between the first and second end effectors, the resection member configured to extend distally beyond the first and second end effectors, the resection member selectively energizable and configured to resect tissue grasped between the first and second end effectors.
2. The surgical device according to paragraph 1, wherein the resection member is selectively deployable from a retracted position, wherein the resection member is disposed within the shaft, to an extended position, wherein the resection member extends distally from the shaft at least partially within the area defined between the first and second end effectors.
3. The surgical device according to paragraph 2, wherein, in the retracted position, the resection member is disposed in a collapsed condition and wherein, in the extended position, the resection member is disposed in an expanded condition.
4. The surgical device according to paragraph 2, wherein, in the extended position, the resection member defines a height greater than heights of the first and second end effectors so as to extend beyond the first and second end effectors in opposing height directions.
5. The surgical device according to paragraph 1, wherein the resection member defines a loop configuration including a leading portion.
6. The surgical device according to paragraph 1, wherein the resection member is pivotable relative to the first and second end effectors through an arcuate path defining a diameter greater than heights of the first and second end effectors.
7. The surgical device according to paragraph 6, wherein the resection member includes a wire defining a semi-circular loop.
8. The surgical device according to paragraph 6, wherein the resection member includes a cup defining a portion of a sphere.
9. The surgical device according to paragraph 6, wherein the resection member defines an elongated configuration including a proximal end portion and a distal end portion, the resection member pivotable about the proximal end portion thereof to move the distal end portion thereof through the arcuate path, the distal end portion including an energizable component.
10. The surgical device according to paragraph 1, wherein each of the first and second end effectors includes first and second jaw members movable relative to one another from a spaced-apart position to an approximated position to grasp tissue therebetween.
11. The surgical device according to paragraph 10, further comprising first and second closure tubes disposed about the first and second end effectors, respectively, the first and second closure tubes movable relative to the first and second end effectors, respectively, to move the first and second jaw members thereof from the spaced-apart position to the approximated position.
12. The surgical device according to paragraph 10, wherein the first and second jaw members of each of the first and second end effectors are coupled to one another via a cam-slot mechanism, and wherein first and second drive rods are operably coupled to the first and second end effectors, respectively, the first and second drive rods movable relative to the first and second end effectors, respectively, to move the first and second jaw members thereof from the spaced-apart position to the approximated position.
13. The surgical device according to paragraph 1, wherein the resection member is adapted to connect to a source of electrosurgical energy.
14. The surgical device according to paragraph 1, wherein the resection member is adapted to connect to a source of laser energy.
15. The surgical device according to paragraph 1, further comprising a housing disposed at a proximal end portion of the shaft, the housing including at least one actuator configured to manipulate the first and second end effectors for grasping tissue therewith.
16. The surgical device according to paragraph 15, wherein the housing further includes at least one second actuator configured to manipulate the resection member.
17. The surgical device according to paragraph 15, wherein the housing further includes an activation button configured to selectively energize the resection member.
18. The surgical device according to paragraph 1, further comprising a robotic arm disposed at a proximal end portion of the shaft, the robotic arm including at least one actuator configured to manipulate the first and second end effectors for grasping tissue therewith.
19. The surgical device according to paragraph 18, wherein the robotic arm further includes at least one second actuator configured to manipulate the resection member.

## Claims

1. A surgical device, comprising:
a shaft;
first and second end effectors extending distally from the shaft in spaced-apart relation relative to one another to define an area therebetween, each of the first and second end effectors including opposing portions movable relative to one another and configured to grasp tissue therebetween; and
a resection member configured for positioning at least partially within the area defined between the first and second end effectors, the resection member configured to extend distally beyond the first and second end effectors, the resection member selectively energizable and configured to resect tissue grasped between the first and second end effectors.

2. The surgical device according to claim 1, wherein the resection member is selectively deployable from a retracted position, wherein the resection member is disposed within the shaft, to an extended position, wherein the resection member extends distally from the shaft at least partially within the area defined between the first and second end effectors.

3. The surgical device according to claim 2, wherein, in the retracted position, the resection member is disposed in a collapsed condition and wherein, in the extended position, the resection member is disposed in an expanded condition.

4. The surgical device according to claim 2 or 3, wherein, in the extended position, the resection member defines a height greater than heights of the first and second end effectors so as to extend beyond the first and second end effectors in opposing height directions.

5. The surgical device according to any preceding claim, wherein the resection member defines a loop configuration including a leading portion.

6. The surgical device according to any preceding claim, wherein the resection member is pivotable relative to the first and second end effectors through an arcuate path defining a diameter greater than heights of the first and second end effectors preferably wherein the resection member includes a wire defining a semi-circular loop or wherein the resection member includes a cup defining a portion of a sphere or wherein the resection member defines an elongated configuration including a proximal end portion and a distal end portion, the resection member pivotable about the proximal end portion thereof to move the distal end portion thereof through the arcuate path, the distal end portion including an energizable component.

7. The surgical device according to any preceding claim, wherein each of the first and second end effectors includes first and second jaw members movable relative to one another from a spaced-apart position to an approximated position to grasp tissue therebetween.

8. The surgical device according to claim 7, further comprising first and second closure tubes disposed about the first and second end effectors, respectively, the first and second closure tubes movable relative to the first and second end effectors, respectively, to move the first and second jaw members thereof from the spaced-apart position to the approximated position.

9. The surgical device according to claim 7, wherein the first and second jaw members of each of the first and second end effectors are coupled to one another via a cam-slot mechanism, and wherein first and second drive rods are operably coupled to the first and second end effectors, respectively, the first and second drive rods movable relative to the first and second end effectors, respectively, to move the first and second jaw members thereof from the spaced-apart position to the approximated position.

10. The surgical device according to any preceding claim, wherein the resection member is adapted to connect to a source of electrosurgical energy.

11. The surgical device according to any preceding claim, wherein the resection member is adapted to connect to a source of laser energy.

12. The surgical device according to any preceding claim, further comprising a housing disposed at a proximal end portion of the shaft, the housing including at least one actuator configured to manipulate the first and second end effectors for grasping tissue therewith.

13. The surgical device according to claim 12, wherein the housing further includes at least one second actuator configured to manipulate the resection member.

14. The surgical device according to claim 12, wherein the housing further includes an activation button configured to selectively energize the resection member.

15. The surgical device according to any preceding claim, further comprising a robotic arm disposed at a proximal end portion of the shaft, the robotic arm including at least one actuator configured to manipulate the first and second end effectors for grasping tissue therewith preferably wherein the robotic arm further includes at least one second actuator configured to manipulate the resection member.
